# EUROPEAN PATENT SPECIFICATION

(11) **EP 2 231 056 B1**
(45) Date of publication and mention of the grant of the patent: **09.03.2016**
(21) Application number: 08866795.1
(22) Date of filing: 24.12.2008
(51) Int. Cl.: A61C 8/00, A61B 17/66

(54) **DENTAL IMPLANT THAT INCREASES BONE SUPPORT**
ZAHNIMPLANTAT, DAS DIE KNOCHENHALTERUNG VERBESSERT
IMPLANT DENTAIRE À ASSISE OSSEUSE RENFORCÉE

(30) Priority: 27.12.2007 TR 200709040
(43) Date of publication of application: 29.09.2010
(73) Proprietor: AKMAN, Serhan, 42075 Konya (TR); TUNALI, Mustafa, 06170 Ankara (TR)
(72) Inventor: AKMAN, Serhan, 42075 Konya (TR); TUNALI, Mustafa, 06170 Ankara (TR)
(74) Representative: 2s | ip Schramm Schneider Patentanwälte Rechtsanwälte
(86) International application number: PCT/TR2008/000145
(87) International publication number: WO 2009/085023

(56) References cited:
- WO-A1-00/21455
- WO-A1-99/37230
- US-A- 5 899 696

## Description

Dental implants are biocompatible functional devices that are placed into bones for substituting an extracted tooth or providing support to fixed or mobile prostheses. The goal of implant design is to provide sufficient initial stability and accomplish osteointegration as early as possible after implantation. Implants are kept load-free for 3 to 6 months following primary surgery. During this period, new regular bone that will stand against the occlusive loads is formed around the implant (osteointegration); so the implant becomes stable enough to resist functional forces created during mastication. Following this stage, prosthetic restoration is done onto the implant to obtain a functional implant.

Magnitude of mechanical stress on alveolar bone depends on the diameter and length of the implant; proper placement of implants with respect to the direction of the forces applied; the number of implants and distribution of the forces. Increasing length of the implant increases its load-bearing surface. Increased length is also crucial for primary stability. Implant should resist shearing and torque forces when abutment is mounted. Increased length also reduces the stress applied on the neck of the implant. Implant body design, screw geometry and surface characteristics are continuously being improved to strengthen the implant-tissue interface, and to attain ideal load transfer; eventually to increase the success and survival of the implant.

High number of anatomic spaces and important nerves in the oral region limits the length of the implants to be used and mandates a detailed examination. If occlusive forces are increased, larger and longer implants should be used. However, confined width and length of the bone restricts the use of implants with appropriate length and width. This negatively affects the long-term success of the implant.

Sagging of the sinus floor, insufficient height and quality of bone are common problems in the patients that have remained toothless for a long time particularly in the posterior maxillary region, and it is rarely possible to place an Implant of sufficient length In proper position. Even if the implant can be placed, this situation causes the implant to have bone support with poor quality. We have inspired from the displacement technique that is based on regeneration of the patient's own bone and widely used in the dentistry for orthodontic procedures to place a long implant to the maxillary posterior region without the need for sinus lifting where bone distance is particularly insufficient; and by using this technique in implantology we aimed to increase the amount of bone around the implant by increasing its length.

US 5,899,696 relates to a dental Implant that can be fixed in an oral cavity, even if the amount of bone required to embed the implant is small. A necessary amount of bone can be secured by generating new bone so that the fixture part of the implant can be fixed in the bone. The fixture part in the implant includes several members movably connected to each other so that the fixture part is adjustable in length. The fixture part includes a first member made up of an uppermost member, middle member, and end member. It is preferred that the middle member be cylindrical or rod-shaped with male screws extending radially outwardly from an outer circumferential periphery thereof.

WO 00/21455 relates to a kit of assemblable components for implantation into the bone of a mammal for use in distraction osteogenesis. The kit comprises a fixture, a footing and a distracter, the fixture including a longitudinally extending body portion with a proximal end and a distal end, the body portion having an exterior surface adapted for contact and integration with bone tissue, the body portion having a generally longitudinally extending bore extending from a proximal opening adjacent the proximal end to a distal opening adjacent the distal end. The footing inclues a proximal surface and a distal surface. The distracter comprises a generally rod-shaped body including a distal end and a proximal end, and the proximal end of the distracter is adapted to bear against the footing. There are first and second engaging means on the fixture and the distracter respectively for adjustably locating the fixture relative to the distracter.

WO 99/37230 relates to a maxillofacial anchoring and distracting system is shown for lengthening the alveolar and small craniofacial skeletal bones by distraction osteogenesis. The distraction system includes a base plug, an internally and externally threaded anchoring screw body, a distraction jack screw and healing screw. The base plug is press-fit into an osteotomy and acts as a baseplate to resist and transfer force received from the distraction jack screw to the subperiosteal corticotomy callus. The application of distraction force to the subperiosteal corticotomy via the distraction jack screw is resisted coronally by the anchoring screw body. Once the desired monofocal distraction osteogenesis has been completed, the maxillofacial anchoring and distraction system can be removed and easily replaced with an optimal length endosseous dental implant.

It is an object of the present invention to provide an implant body for osteogenesis within the maxillary sinus which is completely tight and can easily be adjusted in its pressure force in situ.

This object is solved by the Implant body according to claim 1.

Throughout the normal development of the root of the teeth, increased root length is associated with increased length of alveolar bone. This physiologic and remarkably slow mechanism allows growth of roots to sufficient length without jeopardizing important structures such as maxillary sinus or Inferior alveolar nerve. In a similar manner, the implant we have designed can be gradually lengthened to allow the use of an implant of sufficient length in areas with insufficient bone length.

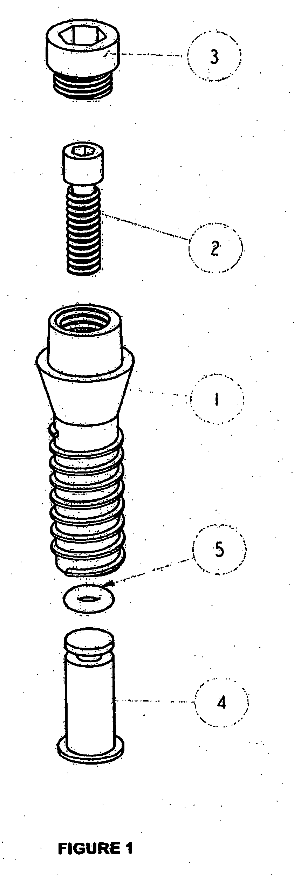

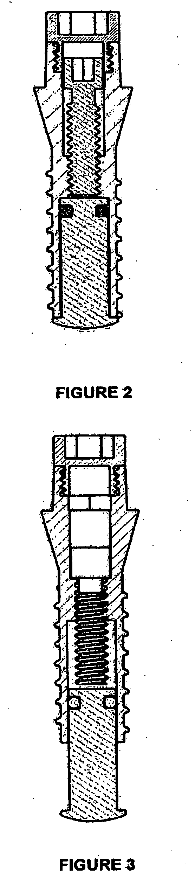

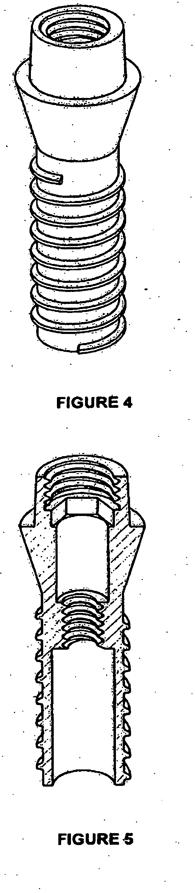

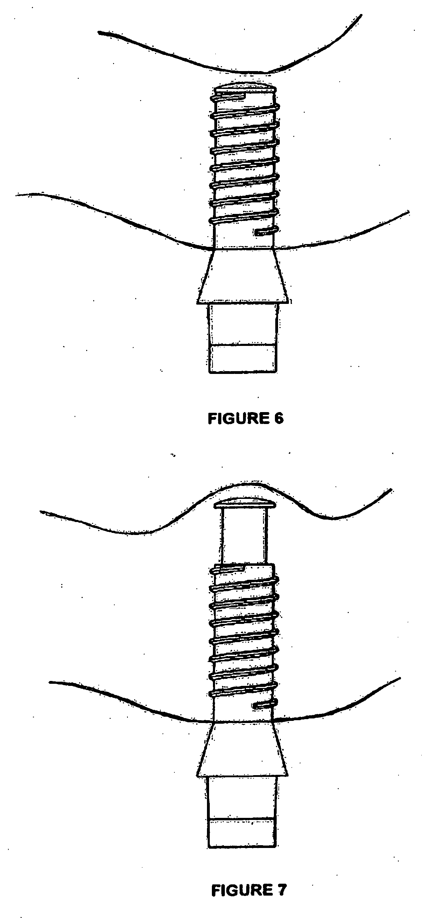

The implant system we have designed consists of 5 parts (Figure 1, 2, 3, 4, 5). In the implant system, there is a pressure adaptor (4) that protrudes and advances through the apical part of the implant body (1). Following insertion of implant system into the posterior maxillary area and attainment of osteointegration (Figure 6), driving screw (2) is used to force the pressure adaptor (4) of the implant system into the floor of the sinus (Figure 7). Force applied within physiologic limits initiates remodeling around the terminal part of the pressure adaptor (4) similar to the behavior of the bones of the teeth that are exposed to orthodontic forces. This process is carried on until a desired implant and bone length is achieved. Implant system also contains a leak-proof element (5) that prevents leakage between pressure adaptor and the implant body. A screw cap (3) is mounted on the implant body to prevent immigration of bacteria and foreign bodies from the oral cavity. These all lead to significant bone formation around the implant system within the maxillary sinus floor. Floor of maxillary sinus is elevated (Figure 7). New bone is formed without the need for an additional surgical procedure.

Our newly developed implant system provides new bone formation by bone remodeling (Figure 6, 7). Unlike the implant distractors that make use of distraction osteogenesis by forming fracture on the bone and make use of flexible fracture callus to form new bone, this implant system make use of remodeling without creating a fracture. Bone formation through remodeling is much slower. This implant system offers a higher patient acceptance as it requires no additional surgery and is less traumatic compared to distractors.

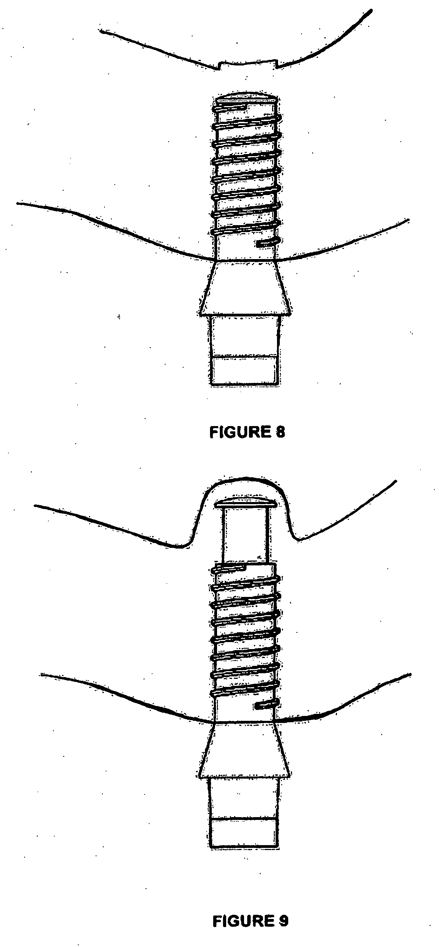

However, by unique application different from the other distractors, this implant system can also be used for distraction osteogenesis in maxillary sinus or other anatomic spaces (Figure 8, 9). Unlike other distractor implants which require osteotomy at the middle part of the implant, in this system only a minimal fracture is formed at the cortical bone above the apical part of the implant as presented in Figure 8 to form callus and consequently new bone in a way similar to distraction osteogenesis. For this procedure, driving screw (2) is advanced and pressure adaptor (4) forces fractured part through the anatomic space fairly faster compared to remodeling (Figure 9). In the distractor implants osteotomy line is at the middle part of the implant.

## Claims

1. Dental implant usable to form new bone and to increase the bone support at the apex of the implant system when placed in the sinus floor or in other anatomic spaces or areas adjacent to anatomic spaces by making use of principles of remodeling and distraction osteogenesis; consisting of the following five distinct parts:
an implant body (1), a pressure adaptor (4) that are in direct contact with the bone, and a driving screw (2), an implant cap (3), and a leak-proof element (5) that are not in direct contact with the bone,
the implant cap (3) being mounted on the implant body (1) to prevent intrusion of bacteria and foreign bodies from oral cavity, and
the leak-proof element (5) being adapted to prevent leakage between the pressure adaptor (4) and the implant body (1),
whereby
the pressure adaptor (4) is adapted to protrude and advance through the apical part of the implant body (1), and
the driving screw (2) is adapted to propel the pressure adaptor (4) through the apical end of the implant body (1) without causing any rotational movement of said pressure adaptor (4).

## Patentansprüche

1. Zahnimplantat, verwendbar, um neuen Knochen zu bilden und um die Knochenstützung an dem Apex des Implantatsystems zu verbessern, wenn in dem Sinusboden oder in anderen anatomischen Räumen oder Gebieten benachbart zu anatomischen Räumen platziert, durch Anwenden von Prinzipien des Remodellierens und der Distraktionsostheogenese; bestehend aus den fünf folgenden verschiedenen Teilen:
ein Implantatkörper (1), ein Druckadapter (4), die in direktem Kontakt zu dem Knochen sind, und eine Treibschraube (2), eine Implantatskappe (3) und ein leckdichtes Element (5), die nicht in direktem Kontakt zu dem Knochen sind,
wobei die Implantatskappe (3) auf dem Implantatskörper (1) montiert ist, um Eindringen von Bakterien und Fremdkörpern von der oralen Kavität zu verhindern, und
wobei das leckdichte Element (5) angepasst ist, Undichtigkeit zwischen dem Druckadapter (4) und dem Implantatkörper (1) zu verhindern,
wobei
der Druckadapter (4) angepasst ist, vorzustehen und vorzudringen durch den apikalen Teil des Implantatkörpers (1), und
die Treibschraube (2) angepasst ist, den Druckadapter (4) durch das apikale Ende des Implantatkörpers (1) zu treiben, ohne jegliche Drehbewegung des Druckadapters (4) zu verursachen.

## Revendications

1. Implant dentaire utilisable pour former de nouvel os et d'accroître le soutien de l'os au sommet du système d'implant lorsqu'il est placé dans le plancher du sinus ou dans d'autres espaces ou des zones adjacentes à des espaces anatomiques, en faisant usage de principes de remodelage et la distraction ostéogenèse anatomiques; constitué par les cinq parties distinctes suivantes:
un corps d'implant (1), un adaptateur de pression (4) qui sont en contact direct avec l'os, et une vis d'entraînement (2), un capuchon d'implant (3), et un élément étanche (5) qui ne sont pas en contact direct avec l'os,
le capuchon d'implant (3) étant monté sur le corps d'implant (1) pour empêcher l'intrusion de bactéries de corps étrangers de la cavité buccale, et
l'élément étanche (5) étant adaptée pour empêcher toute fuite entre l'adaptateur de pression (4) et le corps d'implant (1),
l'adaptateur de pression (4) étant adapté pour faire saillie et avancer à travers la partie apicale du corps d'implant (1), et
la vis d'entraînement (2) étant adaptée pour propulser l'adaptateur de pression (4) à travers l'extrémité apicale du corps d'implant (1) sans provoquer de mouvement de rotation dudit adaptateur de pression (4).
